# EUROPEAN PATENT SPECIFICATION

(11) **EP 1 718 275 B1**
(45) Date of publication and mention of the grant of the patent: **25.07.2007**
(21) Application number: 05713302.7
(22) Date of filing: 09.02.2005
(51) Int. Cl.: A61K 9/00

(54) **CAPILLARY MODERATOR IN AN OSMOTIC DELIVERY SYSTEM FOR PREVENTING BACKFLOW INTO THE ACTIVE AGENT RESERVOIR**
KAPILLARMODERATOR IN EINEM OSMOTISCHEN ABGABESYSTEM ZUR PRÄVENTION VON RÜCKFLUSS IN DAS WIRKSTOFFRESERVOIR
MODERATEUR CAPILLARE DANS UN SYSTEME DE DISTRIBUTION OSMOTIQUE POUR EMPECHER UN REFOULEMENT DANS LE RESERVOIR D'AGENT ACTIF

(30) Priority: 10.02.2004 US 543423 P; 07.02.2005 US 52382
(43) Date of publication of application: 08.11.2006
(73) Proprietor: ALZA CORPORATION, Mountain View, CA 94039-7210 (US)
(72) Inventor: PAN, Li-Wei, Fremont, CA 94539 (US); PANOS, Rodney, M., Redwood City, CA 94062 (US); GOLDMAN, Eli, Joseph, San Francisco, CA 94115 (US)
(74) Representative: Williams, Paul Edwin
(86) International application number: PCT/US2005/004277
(87) International publication number: WO 2005/077334

(56) References cited:
- EP-A- 0 254 394
- WO-A-03/024357
- US-A- 3 760 806

## Description

### CROSS-REFERENCE TO RELATED APPLICATIONS

This application claims benefit to U.S. Provisional Application No. 60/543,423, filed February 10, 2004, and U.S. Patent Application Serial No. 11/052,382 filed February 7, 2005, the entirety of which are incorporated by reference herein.

### FIELD OF THE INVENTION

The present invention relates to apparatus and methods for preventing backflow into a beneficial agent dispensing osmotic delivery system.

### BACKGROUND OF THE INVENTION

Relatively long term controlled delivery of beneficial agents can be accomplished by a variety of methods. One excellent method involves the use of an implantable osmotic delivery system ("ODS"). In general, ODSs operate by taking in fluid from the surrounding environment through one port and releasing corresponding amounts of the beneficial agent from another port ("exit port"). Pressure is generated by an osmotic pump, typically a water-attracting agent, which causes a reliable and constant delivery rate of the beneficial agent from the exit port.

Ideally, the exit port should prevent diffusion or reflux backflow of external fluids into the ODS, as external fluids may adversely affect the utility of the beneficial agent, such as by contaminating, destabilizing, diluting, or otherwise altering the beneficial agent formulation. Moreover, backflow can deleteriously affect the beneficial agent delivery rate in a number of ways. Furthermore, external fluids may cause clogging of the exit port, which can also deleteriously affect the beneficial agent delivery rate.

Backflow at exit ports has been addressed by the addition of slit orifices (see U.S. Patent No. 6,217,906) or sliding pistons (see U.S. 6,508,808), as well as flow moderators with a single exit channel. Systems with a long straight exit channel are impractical for implantation applications because they increase the size of the implant significantly. As it is desirable that implants have as small a profile as possible, flow moderators with a relatively short axial dimension are valuable. In the past, single exit channels were wound through a housing to generate a sufficient passage length to discourage inward flux of materials, but this understandably requires relatively complicated manufacturing conditions.

EP0254394 discloses a dosage form, in particular an osmotic dispensing device, for delivering a beneficial drug formulation to a biological environment of use. This presentation comprises a) a wall which surrounds and forms a compartment, b) a dosage amount of the beneficial agent in the compartment and c) exit means - composed of a plurality of micropores - in the wall of the dosage form. A release rate controlling film surrounding a buffer or osmagent helps to control the solubility of the drug formulation in the presence of external fluid imbibed into the compartment. The exit orifice does not seem to control or prevent the backflow from the external surrounding into the internal compartment.

In WO03024357 a drug delivery device is shown comprising a) a capsule for implantation into the body and comprising a reservoir for a substance, at least one port for allowing said substance to diffuse from or otherwise exit said reservoir, b) a nanopore membrane in communication with said capsule controlling the rate of diffusion of said substance from said at least one exit port, and c) an osmotic engine for affecting the flow through the device.

US3760806 focusses on an osmotic active agent dispenser. The dispensing head can be provided with a check valve (a one way ball valve) to prevent backflow of active agent or other materials from the external environment into the device.

It has now been discovered that backflow can be controlled by the addition of capillary moderators of the present invention to the exit port.

### SUMMARY OF THE INVENTION

Osmotic delivery systems for dispensing beneficial agents are described, comprising a housing having an inlet and an outlet, a beneficial agent reservoir disposed in the housing, and capillary moderators disposed in the outlet for preventing backflow into the beneficial agent reservoir. In one embodiment, the capillary moderators contain hydrophobically coated micro channels.

A method is described for preventing backflow into the beneficial agent reservoir of an osmotic delivery system, comprising providing a capillary moderator between the environment and the beneficial agent reservoir.

### BRIEF DESCRIPTION OF THE DRAWINGS

Fig. 1 is a schematic sectional view of an implantable osmotic delivery system.

Fig. 2 is a schematic perspective view of a capillary moderator of the present invention.

Fig. 3A is a digital image of a SEM micrograph of a capillary moderator of the present invention.

Fig. 3B is a digital image of a SEM micrograph of a crenulated micro channel of the present invention.

Fig. 4 is a schematic view of a micro channel of the present invention.

Fig. 5 shows a plot of pressure versus flow rate for 50µm coated micro channels.

### DETAILED DESCRIPTION OF ILLUSTRATIVE EMBODIMENTS

The present invention relates to apparatus and methods for preventing backflow into a beneficial agent dispensing osmotic delivery system.

An osmotic delivery system 10 comprises a housing 12. The housing 12 may be made any material sufficiently rigid to withstand expansion of the its contents without changing size or shape. It is understood that the housing 12 is impermeable to fluids and gases typically found in vivo.

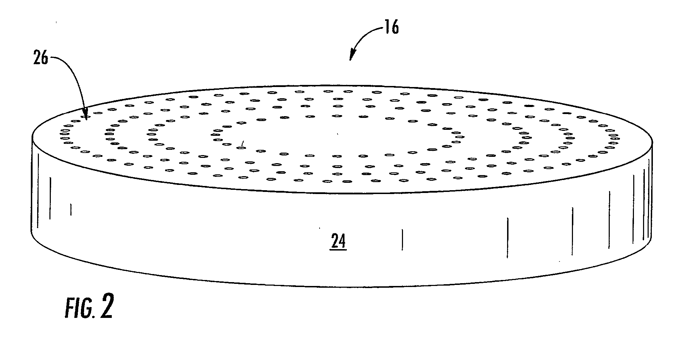

An inlet port 14 and an exit port 16 are disposed in housing 12. The inlet port 14 may include a semi-permeable membrane for allowing fluid to enter the housing 12. As will be discussed in more detail with reference to Fig. 2, the exit port 16 includes hydrophobically coated micro channels.

A piston 18 is slidably disposed in the housing 12, and divides the housing to seal between and define two chambers, namely, a pump chamber 20 and a delivery chamber 22. The pump chamber 20 receives an osmotic agent which swells upon contact with water. The osmotic agent may be, for example, a non-volatile water soluble osmagent, or an osmopolymer, or a mixture thereof. Upon swelling, the osmotic agent exerts a force, which moves the piston 18 towards the exit port 16, thereby increasing the pressure in the delivery chamber 22.

The delivery chamber 22 receives a beneficial agent to be delivered. Increasing pressure from the piston 18 dispenses the beneficial agent out the exit port 16 and into the environment.

According to other embodiments of the present invention, the system 10 may take different forms. For example, the piston 18 may be replaced with a flexible member such as a diaphragm, partition, pad, flat sheet, spheroid, or rigid metal alloy, and may be made of any number of inert materials. Furthermore, the system 10 may function without the piston, having simply an interface between the osmotic agent/fluid additive and the beneficial agent.

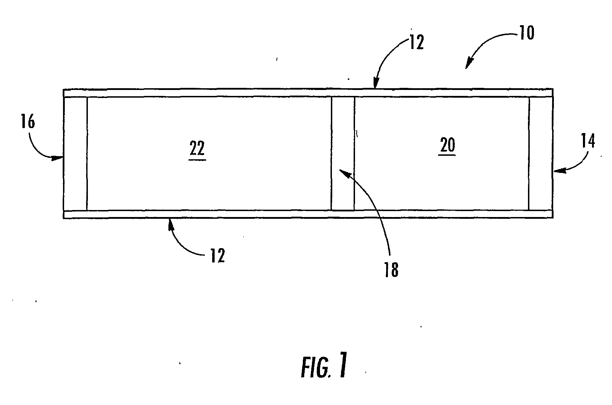

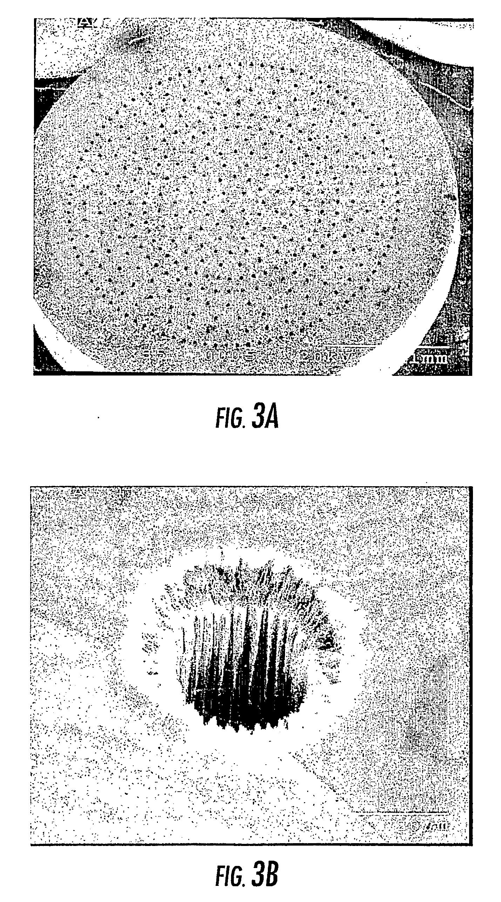

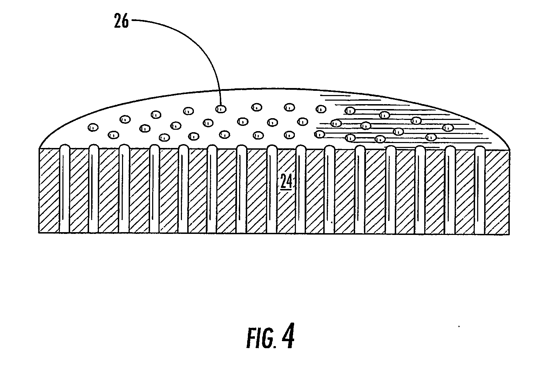

Turning to Figs. 2-4, the exit port 16 (Fig. 1) comprises a capillary moderator 24, having a plurality of micro channels 26. The capillary moderator 24 has a plurality of micro channels 26. The micro channels 26 extend through the capillary moderator 24, and are disposed in an array. The micro channels 26 prevent fluids from flowing from the environment to the beneficial agent reservoir.

In general, the micro channels 26 have a diameter in a range from about 10 µm to about 100 µm. Preferably, the micro channels 26 have a diameter in a range from about 15 µm to about 50 µm. More preferably, the micro channels 26 have a diameter selected from about 15 µm, about 30 µm, and about 50 µm.

The micro channels 26 extend through the capillary moderator 24, so that the length of the micro channels depends on the thickness of the capillary moderator. In one embodiment, the micro channels 26 have a length in a range from about 150 µm to about 400 µm. Preferably, the micro channels 26 have a length of about 300 µm.

In one embodiment, the micro channels 26 are circular in cross section. Though not wishing to be bound by theory, it is believed that the circular cross section maximizes the edge to area ratio in the cross section of channels, such that the effects of the fluid's surface energy are maximized.

In one embodiment, the micro channels 26 are crenulated.

In one embodiment, the micro channels 26 are coated with a polymer or mix thereof that has interfacial tension less than 30 dyn/cm at 20oC, which would provide a low surface energy. Preferably, the polymer would be capable of being made into a gas and applied by conventional plasma coating.

In one embodiment, the micro channels 26 are coated with a hydrophobic polymer, preferably a hydrophobic fluropolymer.

In one embodiment, the micro channels 26 are coated with one or more of Poly (1,1-dihydro-perfluorooctyl methacrylate), Poly(hexafluoropropylene), Poly(tetrafluoroethylene), Poly(vinylidene fluoride), Poly(1,2-butadiene), Polyisobutylene, Poly(vinyl fluoride), Poly(vinyl methyl ether), Polypropylene), Poly(t-butylstyrene), Halogenated Hydrocarbons, including Poly(hexafluoroethylene) and Poly(tetrafluoroethylene), Vinyl Polymers, including Poly((heptafluoroisopropoxy)ethylene), Nonfluorinated Acrylic Polymers, including Poly(ethyl acrylate), Fluorinated Acrylic Polymers, including Poly((1-chlorodifluoromethyl)tetrafluoroethyl acrylate)), Poly(di(chlorodifluoromethyl)fluoromethyl acrylate), Poly(1,1-dihydroheprafluorobutyl acrylate), Poly(1, 1-dihydropentafluoroisoprpyl acrylate), Poly(1, 1-dihydropentadecafluorooctyl acrylate), Poly(heptafluoroisopropyl acrylate), Poly(5-(heptafluoroisopropoxy)pentyl acrylate), Poly(11-(heptafluoroisopropoxy)ethyl acrylate), Poly(2-heptafluoropropoxy)ethyl acrylate, and Poly(nonafluoroisobutyl acrylate), Nonfluorinated Methacrylic Polymers, including Poly(isobutyl methacrylate) and Poly(t-butyl methacrylate), Fluorinated Methacrylic Polymers, including Poly(1,1-dihydropentadecafluorooctyl methacrylate), Poly(heptadecafluorooctyl methacrylate), Poly(heptafluoroisopropyl methacrylate), Poly(1-hydrotetrafluoroethyl methacrylate), Poly(1,1-dihydrotetrafluoropropyl methacrylate), Poly(1-hydrohexafluoroisopropyl methacrylate), Poly(t-nonafluorobutyl methacrylate), Polyethers, including Poly(oxyisobutene)-diol, Poly(imines), including Poly((benzoylimino)ethylene), Poly((butylrylimino)ethylene), Poly(dodecanoylimino)ethylene), Poly((heptanoylimino)ethylene), Poly((hexanoylimino)ethylene), Poly(((3-methyl)butyrylimino)ethylene), Poly((pentadecafluorooctadecanoylimino)ethylene), and Poly((pentanoylimino)ethylene), or Poly(siloxanes), including Poly(oxydiethylsilylene) and Poly(oxydimethylsilylene).

In one embodiment, the polymer is applied by conventional plasma coating. The thickness of the coating in the micro channels varies in a range from about 0.50 µm to about 2 µm, and is preferably about 1 µm.

In one embodiment, fabrication of the moderator 24 starts with a 4" silicon wafer having a 300µm in thickness. The wafer is then cleaned with piranha clean. A 7µm thick positive photoresist is spin-coated on the wafer. A mask is used to pattern areas where micro channels are desired. The micro channels are etched through the wafer by applying DRIE (Deep Reactive Ion Etching). Next, the wafer is treated with oxygen plasma to remove photoresist and clean up the surface. A fluoropolymer plasma treatment, such as can be performed by 4th State, Inc., Belmont, California, USA, is used to coat the wafer surfaces, including the micro channels.

Materials which may be used for the housing 12 should be sufficiently strong to ensure that the housing will not leak, crack, break, or distort under stresses to which they would be subjected during implantation or under stresses due to the pressures generated during operation. The housing 12 may be formed of chemically inert and biocompatible, natural or synthetic materials which are known in the art. The material of the housing 12 is preferably a non-bioerodible material which remains in the patient after use, such as titanium. However, the material of the housing 12 may alternatively be of bioerodible material which bioerodes in the environment after dispensing of the beneficial agent. Generally, preferred materials for the housing 12 are those acceptable for human implants. In general, typical materials of construction suitable for the housing 12 according to the present invention include non-reactive polymers or biocompatible metals or alloys. The polymers include acrylonitrile polymers such as acrylonitrile-butadiene-styrene terpolymer, and the like; halogenated polymers such as polytetrafluoroethylene, polychlorotrifluoroethylene, copolymer of tetrafluoroethylene and hexafluoropropylene; polyimide; polysulfone; polycarbonate; polyethylene; polypropylene; polyvinylchloride-acrylic copolymer; polycarbonate-acrylonitrile-butadiene-styrene; polystyrene; and the like. Metallic materials useful for the housing 12 include stainless steel, titanium, platinum, tantalum, gold, and their alloys, as well as gold-plated ferrous alloys, platinum-plated ferrous alloys, cobalt-chromium alloys and titanium nitride coated stainless steel.

In general, materials suitable for use in the piston 18 are elastomeric materials including the non-reactive polymers listed above, as well as elastomers in general, such as polyurethanes and polyamides, chlorinated rubbers, styrene-butadiene rubbers, and chloroprene rubbers.

The osmotic agent may be a tablet which is a fluid-attracting agent used to drive the flow of the beneficial agent. The osmotic agent may be an osmagent, an osmopolymer, or a mixture of the two. Species which fall within the category of osmagent, i.e., the non-volatile species which are soluble in water and create the osmotic gradient driving the osmotic inflow of water, vary widely. Examples are well known in the art and include magnesium sulfate, magnesium chloride, potassium sulfate, sodium chloride, sodium sulfate, lithium sulfate, sodium phosphate, potassium phosphate, d-mannitol, sorbitol, inositol, urea, magnesium succinate, tartaric acid, raffinose, and various monosaccharides, oligosaccharides and polysaccharides such as sucrose, glucose, lactose, fructose, and dextran, as well as mixtures of any of these various species. Species which fall within the category of osmopolymer are hydrophilic polymers that swell upon contact with water, and these vary widely as well. Osmopolymers may be of plant or animal origin, or synthetic, and examples of osmopolymers are well known in the art. Examples include: poly(hydroxy-allyl methacrylates) with molecular weight of 30,000 to 5,000,000, poly(vinylpyrrolidone) with molecular weight of 10,000 to 360,000, anionic and cationic hydrogels, polyelectrolyte complexes, poly(vinyl alcohol) having low acetate residual, optionally cross-linked with glyoxal, formaldehyde or glutaraldehyde and having a degree of polymerization of 200 to 30,000, a mixture of methyl cellulose, cross-linked agar and carboxymethylcellulose, a mixture of hydroxypropyl methylcellulose and sodium carboxymethylcellulose, polymers of N-viliyllactams, polyoxyethylene-polyoxypropylene gels, polyoxybutylene-polyethylene block copolymer gels, carob gum, polyacrylic gels, polyester gels, polyurea gels, polyether gels, polyamide gels, polypeptide gels, polyamino acid gels, polycellulosic gels, carbopol acidic carboxy polymers having molecular weights of 250,000 to 4,000,000, Cyanamer polyacrylamides, cross-linked indene-maleic anhydride polymers, Good-Rite polyacrylic acids having molecular weights of 80,000 to 200,000, Polyox polyethylene oxide polymers having molecular weights of 100,000 to 5,000,000, starch graft copolymers, and Aqua-Keeps acrylate polymer polysaccharides.

In one embodiment of this invention, the beneficial agents contained in the chamber 22 are flowable compositions such as liquids, suspension, or slurries, and are poured into the housing 12 after the osmotic agent and the piston 18 have been inserted. Alternatively, such flowable compositions may be injected with a needle through a slit in the port, which allows for filling without air bubbles. The present invention applies to the administration of beneficial agents in general, which include any physiologically or pharmacologically active substance. The beneficial agent may be any of the agents which are known such as drug agents, medicaments, vitamins, nutrients, or the like. The beneficial agent may also be an agent which is delivered to other types of aqueous environments such as pools, tanks, reservoirs, and the like. Included among the types of agents which meet this description are biocides, sterilization agents, nutrients, vitamins, food supplements, sex sterilants, fertility inhibitors and fertility promoters. Drug agents which may be delivered by the present invention include drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable agents may be selected from, for example, proteins, enzymes, hormones, polynucleotides, nucleoproteins, polysaccharides, glycoproteins, lipoproteins, polypeptides, steroids, analgesics, local anesthetics, antibiotic agents, anti-inflammatory corticosteroids, ocular drugs and synthetic analogs of these species. Examples of drugs which may be delivered by systems according to this invention include, but are not limited to prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, digoxin, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17-hydroxyprogesterone acetate, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, milrinone, capropril, mandol, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat, captopril, ramipril, famotidine, nizatidine, sucralfate, etintidine, tetratolol, minoxidil, chlordiazepoxide, diazepam, amitriptyline, and imipramine. Further examples are proteins and peptides which include, but are not limited to, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, prolactin, somatostatin, lypressin, pancreozymin, luteinizing hormone, LHRH, LHRH agonists and antagonists, leuprolide, interferons, interleukins, growth hormones such as human growth hormone, bovine growth hormone and porcine growth hormone, fertility inhibitors such as the prostaglandins, fertility promoters, growth factors, coagulation factors, human pancreas hormone releasing factor, analogs and derivatives of these compounds, and pharmaceutically acceptable salts of these compounds, or their analogs or derivatives. The beneficial agent can be present in this invention in a wide variety of chemical and physical forms, such as solids, liquids and slurries. On the molecular level, the various forms may include uncharged molecules, molecular complexes, and pharmaceutically acceptable acid addition and base addition salts such as hydrochlorides, hydrobromides, sulfate, laurylate, oleate, and salicylate. For acidic compounds, salts of metals, amines or organic cations may be used. Derivatives such as esters, ethers and amides can also be used. An active agent can be used alone or mixed with other active agents.

For the administration of beneficial agents, the systems of the present invention may be implanted subcutaneously or intraperitoneally or at any other location in a biological environment where aqueous body fluids are available to activate the osmotic engine. The systems of this invention are also useful in environments outside of physiological or aqueous environments. For example, the systems may be used in intravenous systems (attached to an IV pump or bag or to an IV bottle, for example) for delivering beneficial agents. They may also be utilized in blood oxygenators, kidney dialysis and electrophoresis, for example. Additionally, systems of the present invention may be used in the biotechnology area, such as to deliver nutrients or growth regulating compounds to cell cultures.

### EXAMPLES

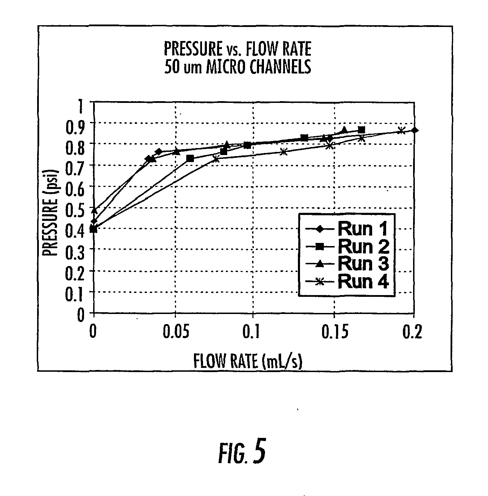

In testing, moderators of the present invention with micro channels of 50µm and 30µm successfully stopped outside pressures of 0.4psi and 0.8psi. Fig. 5 shows a plot of pressure versus flow rate for 50 µm coated micro channels.

The disclosures of each patent, patent application, and publication cited or described in this document are hereby incorporated herein by reference, in their entireties.

Each recited range includes all combinations and subcombinations of ranges, as well as specific numerals contained therein.

Various modifications of the invention, in addition to those described herein, will be apparent to those skilled in the art from the foregoing description. Such modifications are also intended to fall within the scope of the appended claims.

## Claims

1. An osmotic delivery system for dispensing a beneficial agent, comprising:
a housing having an inlet and an outlet;
a beneficial agent reservoir disposed in the housing; and
a capillary moderator disposed in the outlet for preventing backflow into the beneficial agent reservoir.

2. The system of claim 1, further comprising an osmotic pump adapted to cause the controlled release of the beneficial agent.

3. The system of claim 1, wherein the capillary moderator has a plurality of micro channels disposed between the environment and the beneficial agent reservoir.

4. The system of claim 3, wherein the micro channels prevent fluids from flowing from the environment to the beneficial agent reservoir.

5. The system of claim 3, wherein the micro channels have a diameter in a range from 10 µm to 100 µm.

6. The system of claim 3, wherein the micro channels have a diameter in a range from 15 µm to 50 µm.

7. The system of claim 3, wherein the micro channels have a diameter selected from about 15 µm, about 30 µm, and about 50 µm.

8. The system of claim 3, wherein the micro channels have a length in a range from 150 um to 400 µm.

9. The system of claim 3, wherein the micro channels have a length of about 300 µm.

10. The system of claim 3, wherein the micro channels are crenulated.

11. The system of claim 3, wherein the micro channels are coated with a hydrophobic polymer.

12. The system of claim 3, wherein the micro channels are coated with a hydrophobic fluropolymer.

13. The system of claim 11, wherein the polymer is selected from at least one of Poly(1,1-dihydro-perfluorooctyl methacrylate), Poly(hexafluoropropylene), Poly(tetrafluoroethylene), Poly(vinylidene fluoride), Poly(1,2-butadiene), Polyisobutylene, Poly(vinyl fluoride), Poly(vinyl methyl ether), Polypropylene), Poly(t-butylstyrene), Halogenated Hydrocarbons, including Poly(hexafluoroethylene) and Poly(tetrafluoroethylene), Vinyl Polymers, including Poly((heptafluoroisopropoxy)ethylene), Nonfluorinated Acrylic Polymers, including Poly(ethyl acrylate), Fluorinated Acrylic Polymers, including Poly((1-chlorodifluoromethyl)tetrafluoroethyl acrylate)), Poly(di(chlorodifluoromethyl)fluoromethyl acrylate), Poly(1,1-dihydroheprafluorobutyl acrylate), Poly(1, 1-dihydropentafluoroisoprpyl acrylate), Poly(1, 1-dihydropentadecafluorooctyl acrylate), Poly(heptafluoroisopropyl acrylate), Poly(5-(heptafluoroisopropoxy)pentyl acrylate), Poly(11-(heptafluoroisopropoxy)ethyl acrylate), Poly(2-heptafluoropropoxy)ethyl acrylate, and Poly(nonafluoroisobutyl acrylate), Nonfluorinated Methacrylic Polymers, including Poly(isobutyl methacrylate) and Poly(t-butyl methacrylate), Fluorinated Methacrylic Polymers, including Poly(1,1-dihydropentadecafluorooctyl methacrylate), Poly(heptadecafluorooctyl methacrylate), Poly(heptafluoroisopropyl methacrylate), Poly(1-hydrotetrafluoroethyl methacrylate), Poly(1,1-dihydrotetrafluoropropyl methacrylate), Poly(1-hydrohexafluoroisopropyl methacrylate), Poly(t-nonafluorobutyl methacrylate), Polyethers, including Poly(oxyisobutene)-diol, Poly(imines), including Poly((benzoylimino)ethylene), Poly((butylrylimino)ethylene), Poly(dodecanoylimino)ethylene), Poly((heptanoylimino)ethylene), Poly((hexanoylimino)ethylene), Poly(((3-methyl)butyrylimino)ethylene), Poly((pentadecafluorooctadecanoylimino)ethylene), and Poly((pentanoylimino)ethylene), and Poly(siloxanes), including Poly(oxydiethylsilylene) and Poly(oxydimethylsilylene).

14. The system of claim 12, wherein the thickness of the coating in the micro channels varies in a range from 0.50 µm to 2 µm.

15. The system of claim 12, wherein the thickness of the coating in the micro channels is about 1 µm.

16. A method for preventing backflow into the beneficial agent reservoir of an osmotic delivery system, comprising:
providing a capillary moderator between the environment and the beneficial agent reservoir.

17. The method of claim 16, wherein the capillary moderator has a plurality of micro channels extending therethrough.

18. The method of claim 17, wherein the micro channels are crenulated.

19. The method of claim 17, further comprising coating the micro channels with a hydrophobic polymer.

20. The method of claim 19, wherein the polymer is selected from at least one of Poly (1,1-dihydro-perfluorooctyl methacrylate), Poly(hexafluoropropylene), Poly(tetrafluoroethylene), Poly(vinylidene fluoride), Poly(1,2-butadiene), Polyisobutylene, Poly(vinyl fluoride), Poly(vinyl methyl ether), Polypropylene), Poly(t-butylstyrene), Halogenated Hydrocarbons, including Poly(hexafluoroethylene) and Poly(tetrafluoroethylene), Vinyl Polymers, including Poly((heptafluoroisopropoxy)ethylene), Nonfluorinated Acrylic Polymers, including Poly(ethyl acrylate), Fluorinated Acrylic Polymers, including Poly((1-chlorodifluoromethyl)tetrafluoroethyl acrylate)), Poly(di(chlorodifluoromethyl)fluoromethyl acrylate), Poly(1,1-dihydroheprafluorobutyl acrylate), Poly(1, 1-dihydropentafluoroisoprpyl acrylate), Poly(1, 1-dihydropentadecafluorooctyl acrylate), Poly(heptafluoroisopropyl acrylate), Poly(5-(heptafluoroisopropoxy)pentyl acrylate), Poly(11-(heptafluoroisopropoxy)ethyl acrylate), Poly(2-heptafluoropropoxy)ethyl acrylate, and Poly(nonafluoroisobutyl acrylate), Nonfluorinated Methacrylic Polymers, including Poly(isobutyl methacrylate) and Poly(t-butyl methacrylate), Fluorinated Methacrylic Polymers, including Poly(1,1-dihydropentadecafluorooctyl methacrylate), Poly(heptadecafluorooctyl methacrylate), Poly(heptafluoroisopropyl methacrylate), Poly(1-hydrotetrafluoroethyl methacrylate), Poly(1,1-dihydrotetrafluoropropyl methacrylate), Poly(1-hydrohexafluoroisopropyl methacrylate), Poly(t-nonafluorobutyl methacrylate), Polyethers, including Poly(oxyisobutene)-diol, Poly(imines), including Poly((benzoylimino)ethylene), Poly((butylrylimino)ethylene), Poly(dodecanoylimino)ethylene), Poly((heptanoylimino)ethylene), Poly((hexanoylimino)ethylene), Poly(((3-methyl)butyrylimino)ethylene), Poly((pentadecafluorooctadecanoylimino)ethylene), and Poly((pentanoylimino)ethylene), and Poly(siloxanes), including Poly(oxydiethylsilylene) and Poly(oxydimethylsilylene).

## Patentansprüche

1. Osmotisches Abgabesystem zum Dispergieren eines nützlichen Mittels, umfassend
ein Gehäuse mit einem Einlaß und einem Abfluß;
einem Reservoir für das nützliche Mittel, das in dem zur Verfügung steht; und
einen Kapillarmoderator, der in dem Abfluß vorhanden ist, um den Rückfluß in das Reservoir mit dem nützlichen Mittel zu verhindern.

2. System nach Anspruch 1, das außerdem eine osmotische Pumpe aufweist, die so angepaßt ist, um die kontrollierte Abgabe des nützlichen Mittels zu bewirken.

3. System nach Anspruch 1, bei dem der Kapillarmoderator eine Vielzahl von Mikrokanälen aufweist, die zwischen der Umgebung und dem Reservoir mit dem nützlichen Mittel angeordnet sind.

4. System nach Anspruch 3, bei dem die Mikrokanäle verhindern, daß Flüssigkeiten aus der Umgebung in das Reservoir mit dem nützlichen Mittel fließen.

5. System nach Anspruch 3, bei dem die Mikrokanäle einen Durchmesser im Bereich von 10 µm bis 100 µm aufweisen.

6. System nach Anspruch 3, bei dem die Mikrokanäle einen Durchmesser im Bereich von 15 µm bis 50 µm aufweisen.

7. System nach Anspruch 3, bei dem die Mikrokanäle einen Durchmesser ausgewählt von etwa 15 µm, etwa 30 µm und etwa 50 µm aufweisen.

8. System nach Anspruch 3, bei dem die Mikrokanäle eine Länge von 150 µm bis 400 µm aufweisen.

9. System nach Anspruch 3, bei dem die Mikrokanäle eine Länge von etwa 300 µm aufweisen.

10. System nach Anspruch 3, bei dem die Mikrokanäle kreneliert sind.

11. System nach Anspruch 3, bei dem die Mikrokänale mit einem hydrophoben Polymer beschichtet sind.

12. System nach Anspruch 3, bei dem die Mikrokänale mit einem hydrophoben Fluorpolymer beschichtet sind.

13. System nach Anspruch 11, bei dem der Polymer wenigstens ausgewählt wird aus Poly(1,1-dihydroperfluoroctylmethacrylat), Poly(hexafluorpropylen), Poly(tetrafluorethylen), Poly(vinylidenfluorid), Poly(1,2-butadien), Polyisobutylen, Poly(vinylfluorid), Poly(vinylmethylether), Polypropylen, Poly(t-butylstyrol), halogenierte Kohlenwasserstoffe, einschließlich Poly(hexafluorethylen) und Poly(tetrafluorethylen), Vinylpolymere, einschließlich Poly((heptafluor-isopropoxy)ethylen), nicht-fluorierte Acrylpolymere, einschließlich Poly(ethylacrylat), fluorierte Acrylpolymere, einschließlich Poly((1-chlordifluormethyl)tetrafluorethylacrylat). Poly(di(chlordifluormethyl)fluormethyl-acrylat). Poly(1,1-dihydroheptanuorbutylacrylat), Poly(1,1-dihydropentafluoriso-propylacrylat). Poly(1.1-dihydropentadecafluoroctyl acrylat), Poly(heptafluoriso-propylacrylat). Poly(5-(heptafluorisopropoxy)-pentylacrylat), Poly(11-(heptafluorisopropoxy)ethylacrylat). Poly(2-heptafluorpropoxy)ethylacrylat, und Poly(nonafluorisobutylacrylat), nicht-fluorierte Methacrylpolymere, einschließlich Poly(isobutylmethacrylat) und Poly(t-butylmethacrylat), fluorierte Methacrylpolymere, einschließlich Poly(1,1-dihydropentadecafluoroctylmeth-acrylat, Poly(heptadecafluoroctylmethacrylat), Poly(heptafluorisopropylmeth-acrylat, Poly(1-hydrotetrafluorethylmethacrylat). Poly(1,1-dihydrotetrafluorpropylmethacrylat). Poly(1-hydrohexafluoriso-propylmethacrylat). Poly(t-nonafluorbutylmethacrylat, Polyether, einschließlich Poly(oxyisobuten)diol, Poly(imine), einschließlich Poly((benzoylimino)ethylen), Poly((butylrylimino)ethylen), Poly(dodecanoylimino)ethylen), Poly((heptanoyl-imino)ethylen), Poly((hexanoylimino)ethylen), Poly(((3-methyl)butyrylimino)ethylen), Poly((pentadecafluoroctadecanoylimino)ethylen, und Poly((pentanoyllmino)-ethlyen), und Poly(siloxane), einschließlich Poly(oxydiethylsilylen) und Poly(oxy-dimethylsllylen).

14. System nach Anspruch 12, bei dem die Dicke der Beschichtung in den Mikrokanälen im Bereich von 0.50 µm bis 2 µm variiert.

15. System nach Anspruch 12, bei dem die Dicke der Beschichtung in den Mikrokanälen etwa 1 µm ist.

16. Verfahren zur Verhinderung des Rückflusses in das Reservoir mit dem nützlichen Mittel eines osmotischen Abgabesystems, umfassend:
Bereitstellung eines Kapillarmoderators zwischen der Umgebung und dem Reservoir mit dem nützlichen Mittel.

17. Verfahren nach Anspruch 16, bei dem der Kapillarmoderator eine Vielzahl von Mikrokanälen aufweist, die sich dadurch erstrecken.

18. Verfahren nach Anspruch 17, bei dem die Mikrokanäle kreneliert sind.

19. Verfahren nach Anspruch 17, bei dem die Mikrokänale mit einem hydrophoben Polymer beschichtet sind.

20. Verfahren nach Anspruch 19, bei dem der Polymer wenigstens ausgewählt wird aus Poly(1,1-dihydroperfluoroctylmethacrylat), Poly(hexafluorpropylen), Poly(tetrafluorethylen). Poly(vinylidenfluorid). Poly(1,2-butadien), Polyisobutylen, Poly(vinylfluorid), Poly(vinylmethylether), Polypropylen, Poly(t-butylstyrol), halogenierte Kohlenwasserstoffe, einschließlich Poly(hexafluorethylen) und Poly(tetrafluorethylen), Vinylpolymere, einschließlich Poly((heptafluor-isopropoxy)ethylen), nicht-fluorierte Acrylpolymere, einschließlich Poly(ethylacrylat), fluorierte Acrylpolymere, einschließlich Poly((1-chlordifluormethyl)tetrafluorethylacrylat). Poly(di(chlordifluormethyl)fluormethyl-acrylat), Poly(1,1-dihydroheptafluorbutylacrylat), Poly(1,1-dihydropentafluoriso-propylacrylat), Poly(1,1-dihydropentadocafluoroctylacrylat), Poly(heptafluoriso-propylacrylat), Poly(5-(heptafluorisopropoxy)pentylacrylat). Poly(11-(heptafluorisopropoxy)ethylacrylat), Poly(2-heptafluorpropoxy)ethylacrylat, und Poly(nonafluorisobutylacrylat), nicht-fluorierte Methacrylpolymere, einschließlich Poly(isobutylmethacrylat) und Poly(t-butylmethacrylat), fluorierte Methacrylpolymere, einschließlich Poly(1,1-dihydropentadecafluoroctylmeth-acrylat, Poly(heptadecafluoroctylmethacrylat), Poly(heptafluorisopropylmeth-acrylat. Poly(1-hydrotetrafluorethylmethacrylat), Poly(1,1-dihydrotetrafluorpropylmethacrylat), Poly(1-hydrohexafluoriso-propylmethacrylat), Poly(t-nonafluorbutylmethacrylat, Polyether, einschließlich Poly(oxyisobuten)diol, Poly(imine), einschließlich Poty((benzoylimino)ethylen). Poly(butylrylimino)ethylen), Poly(dodecanoylimino)ethylen). Poly((heptanoyl-imino)ethylen). Poly((hexanoylimino)ethylen), Poly(((3-methyl)butyrylimino)ethylen). Poly((pentadecafluoroctadecanoylimino)ethylen, und Poly((pentanoylimino)-ethlyen), und Poly(siloxane), einschließlich Poly(oxydiethylsilylen) und Poly(oxy-dimethylsilylen).

## Revendications

1. Système de distribution osmotique pour distribuer un agent utile, comprenant :
- une enveloppe ayant une entrée et une sortie;
- un réservoir d'agent utile disposé dans l'enveloppe; et
- un modérateur capillaire disposé dans la sortie pour empêcher un reflux dans le réservoir d'agent utile.

2. Système selon la revendication 1, comprenant en outre une pompe osmotique adaptée pour provoquer la libération contrôlée de l'agent utile.

3. Système selon la revendication 1, dans lequel le modérateur capillaire a une pluralité de micro canaux disposés entre l'environnement et le réservoir d'agent utile.

4. Système selon la revendication 3, dans lequel les micro canaux empêchent les fluides de s'écouler de l'environnement dans le réservoir d'agent utile.

5. Système selon la revendication 3, dans lequel les micro canaux ont un diamètre se situant dans une plage allant de 10 µm à 100 µm.

6. Système selon la revendication 3, dans lequel les micro canaux ont un diamètre se situant dans une plage allant de 15 µm à 50 µm.

7. Système selon la revendication 3, dans lequel les micro canaux ont un diamètre choisi parmi environ 15 µm, environ 30 µm et environ 50 µm.

8. Système selon la revendication 3, dans lequel les micro canaux ont une longueur se situant dans une plage allant de 150 µm à 400 µm.

9. Système selon la revendication 3, dans lequel les micro canaux ont une longueur d'environ 300 µm.

10. Système selon la revendication 3, dans lequel les micro canaux sont crénelés.

11. Système selon la revendication 3, dans lequel les micro canaux sont revêtus d'un polymère hydrophobe.

12. Système selon la revendication 3, dans lequel les micro canaux sont revêtus d'un fluoropolymère hydrophobe.

13. Système selon la revendication 11, dans lequel le polymère est sélectionné à partir d'au moins l'un parmi le poly(méthacrylate de 1,1-dihydro-perfluorooctyle), le poly(hexafluoropropylène), le poly(tétrafluoroéthylène), le poly(fluorure de vinylidène), le poly(1,2-butadiène), le polyisobutylène, le poly(fluorure de vinyle), le poly(méthyl vinyl éther), le polypropylène, le poly(t-butylstyrène), les hydrocarbures halogénés comprenant le poly(hexafluoroéthylène) et le poly(tétrafluoroéthylène), les polymères vinyliques comprenant le poly((heptafluoroisopropoxy)éthylène), les polymères acryliques non fluorés comprenant le poly(acrylate d'éthyle), les polymères acryliques fluorés comprenant le poly(acrylate de (1-chlorodifluorométhyl) tétrafluoroéthyle), le poly(acrylate de di(chlorodifluorométhyl)fluorométhyle), le poly(acrylate de 1,1-dihydroheptafluorobutyle), le poly(acrylate de 1,1-dihydropentafluoroisopropyle), le poly(acrylate de 1,1-dihydropentadécafluorooctyle), le poly(acrylate d'heptafluoroisopropyle), le poly(acrylate de 5-(heptafluoroisopropoxy)pentyle), le poly(acrylate de 11-(heptafluoroisopropoxy)éthyle), le poly(acrylate de 2-heptafluoropropoxyéthyle) et le poly(acrylate de nonafluoroisobutyle), les polymères méthacryliques non fluorés comprenant le poly(méthacrylate d'isobutyle) et le poly(méthacrylate de t-butyle), les polymères méthacryliques fluorés comprenant le poly(méthacrylate de 1,1-dihydropentadécafluorooctyle), le poly(méthacrylate de heptadécafluorooctyle), le poly(méthacrylate de heptafluoroisopropyle), le poly(méthacrylate de 1-hydrotétrafluoroéthyle), le poly(méthacrylate de 1,1-dihydrotétrafluoropropyle), le poly(méthacrylate de 1-hydrohexafluoroisopropyle), le poly(méthacrylate de t-nonafluorobutyle), les polyéthers comprenant le poly(oxyisobutène)-diol, les poly(imines) comprenant le poly((benzoylimino)éthylène), le poly((butyrylimino)éthylène), le poly((dodécanoylimino)éthylène), le poly((heptanoylimino)éthylène), le poly((hexanoylimino)éthylène), le poly(((3-méthyl)butyrylimino)éthylène), le poly((pentadécafluorooctadécanoylimino)éthylène) et le poly((pentanoylimino)éthylène), et les poly(siloxanes), comprenant le poly(oxydiéthylsilylène) et le poly(oxydiméthylsilylène).

14. Système selon la revendication 12, dans lequel l'épaisseur du revêtement dans les micro canaux varie dans une plage allant de 0,50 µm à 2 µm.

15. Système selon la revendication 12, dans lequel l'épaisseur du revêtement dans les micro canaux est d'environ 1 µm.

16. Procédé pour empêcher un reflux dans le réservoir d'agent utile d'un système de distribution osmotique, comprenant l'opération consistant à :
- disposer un modérateur capillaire entre l'environnement et le réservoir d'agent utile.

17. Procédé selon la revendication 16, dans lequel le modérateur capillaire a une pluralité de micro canaux s'étendant à travers lui.

18. Procédé selon la revendication 17, dans lequel les micro canaux sont crénelés.

19. Procédé selon la revendication 17, comprenant en outre le revêtement des micro canaux par un polymère hydrophobe.

20. Procédé selon la revendication 19, dans lequel le polymère est sélectionné à partir d'au moins l'un parmi le poly(méthacrylate de 1,1-dihydro-perfluorooctyle), le poly(hexafluoropropylène), le poly(tétrafluoroéthylène), le poly(fluorure de vinylidène), le poly(1,2-butadiène), le polyisobutylène, le poly(fluorure de vinyle), le poly(méthyl vinyl éther), le polypropylène, le poly(t-butylstyrène), les hydrocarbures halogénés comprenant le poly(hexafluoroéthylène) et le poly(tétrafluoroéthylène), les polymères vinyliques comprenant le poly((heptafluoroisopropoxy)éthylène), les polymères acryliques non fluorés comprenant le poly(acrylate d'èthyle), les polymères acryliques fluorés comprenant le poly(acrylate de (1-chlorodifluorométhyl) tétrafluoroéthyle), le poly(acrylate de di(chlorodifluorométhyl)fluorométhyle), le poly(acrylate de 1,1-dihydroheptafluorobutyle), le poly(acrylate de 1,1-dihydropentafluoroisopropyle), le poly(acrylate de 1,1-dihydropentadécafluorooctyle), le poly(acrylate d'heptafluoroisopropyle), le poly(acrylate de 5-(heptafluoroisopropoxy)pentyle), le poly(acrylate de 11-(heptafluoroisopropoxy)éthyle), le poly(acrylate de 2-heptafluoropropoxyéthyle) et le poly(acrylate de nonafluoroisobutyle), les polymères méthacryliques non fluorés comprenant le poly(méthacrylate d'isobutyle) et le poly(méthacrylate de t-butyle), les polymères méthacryliques fluorés comprenant le poly(méthacrylate de 1,1-dihydropentadécafluorooctyle), le poly(méthacrylate de heptadécafluorooctyle), le poly(méthacrylate de heptafluoroisopropyle), le poly(méthacrylate de 1-hydrotétrafluoroéthyle), le poly(mèthaarylate de 1,1-dihydrotetrafluoropropyle), le poly(méthacrylate de 1-hydrohexafluoroisopropyle), le poly(méthacrylate de t-nonafluorobutyle), les polyéthers comprenant le poly(oxyisobutène)-diol, les poly(imines) comprenant le poly((benzoylimino)éthylène), le poly((butyrylimino)éthylène), le poly((dodécanoylimino)éthylène), le poly((heptanoylimino)éthylène), le poly((hexanoylimino)éthylène), le poly(((3-méthyl)butyrylimino)éthylène), le poly((pentadécafluorooctadécanoylimino)éthylène) et le poly((pentanoylimino)éthylène), et les poly(siloxanes), comprenant le poly(oxydiéthylsilyène) et le poly(oxydiméthylsilylène).
